# NEUE EUROPÄISCHE PATENTSCHRIFT

(11) **EP 0 734 698 B2**
(45) Veröffentlichungstag und Bekanntmachung des Hinweises auf die Entscheidung über den Einspruch: **29.03.2006**
(45) Hinweis auf die Patenterteilung: 12.08.1998
(21) Anmeldenummer: 96104982.2
(22) Anmeldetag: 28.03.1996
(51) Int. Cl.: A61F 2/06

(54) **Stent zur transluminalen Implantation in Hohlorgane**
Stent for transluminal implantation into hollow organs
Stent pour implantation transluminale dans des organes creux

(30) Priorität: 01.04.1995 DE 19512066; 07.05.1995 DE 19516191; 30.10.1995 DE 19540851
(43) Veröffentlichungstag der Anmeldung: 02.10.1996
(73) Patentinhaber: Variomed AG, 9496 Balzers (LI)
(72) Erfinder:
(74) Vertreter: Manitz, Finsterwald & Partner GbR

(56) Entgegenhaltungen:
- EP-A- 0 335 341
- EP-A- 0 540 290
- EP-A- 0 566 807
- EP-A- 0 709 067
- WO-A-96/03092
- DE-A- 4 303 181
- GB-A- 2 281 865
- US-A- 5 133 732
- US-A- 5 139 480
- US-A- 5 449 373

## Beschreibung

Die vorliegende Erfindung betrifft einen Stent zur transluminalen Implantation in Hohlorgane, insbesondere in Blutgefäße, Harnleiter. Speiseröhren oder Gallenwege mit einem im wesentlichen röhrenförmigen Körper, der von einem komprimierten Zustand mit einem ersten Querschnittsdurchmesser in einen expandierten Zustand mit einem vergrößerten zweiten Querschnittsdurchmesser überführbar ist, wobei die Wand des röhrenförmigen Körpers sich sowohl in Längsrichtung als auch in Umfangsrichtung des Stents wiederholande, die Expansion gewährleistende Durchbrechungen aufweist.

Ein Stent dieser Art ist aus der EP-A-0 335 341 bekannt. Der Stent besteht aus einzelnen röhrenförmigen Elementen, in deren Wänden parallel zur Längsachse des Stents angeordnete Durchbrechungen ausgebildet sind. Die einzelnen Elemente sind über Verbindungsabschnitte miteinander verbunden, wobei in den Wänden der Verbindungsabschnitte Durchbrechungen ausgebildet sind, die jeweils einen schräg und einen parallel zur Längsachse des Stents angeordneten länglichen Abschnitt umfassen und eine Verkippen der röhrenförmigen Elemente gegeneinander ermöglichen.

Ein weiterer Stent ist aus der nachveröffentlichten EP-A-0 709 067, die einen Stand der Technik gemäß Art. 54(3) EPÜ bildet, bekannt Auch dieser Stent besitzt Durchbrechungen, die parallel zur Längsachse des Stents verlaufen und keine schräg zur Längsachse des Stents angeordneten Abschnitte aufweisen.

Stents der eingangs genannten Art werden zur Rekanalisation von krankhaft veränderten Hohlorganen eingesetzt. Dabei werden die Stents im komprimierten Zustand über einen Einführkatheter an die zu behandelnde Stelle innerhalb des Hohlorgans eingeführt, wo sie durch unterschiedliche Maßnahmen auf einen Durchmesser, der dem des gesunden Hohlorgans entspricht, expandiert werden können, so daß eine Stützwirkung des Hohlorgans, beispielsweise einer Gefäßwand, erreicht wird.

Je nachdem, auf welche Weise der expandierte Zustand erreicht wird, unterscheidet man zwischen ballonexpandierten Stents und selbstexpandierenden Stents. Ballonexpandierte Stents werden im komprimierten Zustand auf einen speziellen Ballonkatheter montiert, bis zu der zu behandelnden Stelle des jeweiligen Hohlorgans eingeführt und dort durch Balloninsufflation auf den gewünschten Durchmesser ausgedehnt Durch die plastische Verformung des Stentmaterials erhält der Stent im expandierten Zustand seine Stabilität, so daß eine ausreichende Stützwirkung des Hohlorgans erzielt wird.

Selbstexpandierende Stents werden durch Hilfsmittel, wie beispielsweise membranartige Überzüge in ihrem komprimierten Zustand gehalten und über einen Katheter an die zu behandelnde Stelle innerhalb des Hohlorgans eingeführt. Nach Entfernen des Überzugs dehnen sich diese Stents durch ihre eigene Spannung von selbst auf einen vorbestimmten Durchmesser im Hohlorgan aus, so daß auf diese Weise die Stützung der Wand des Hohlorgans erreicht wird. Grundsätzlich können auch diese selbstexpandierenden Stents mit Hilfe eines Ballonkatheters an die Gefäßwand nachgedrückt werden.

Zu der Gruppe der selbstexpandierenden Stents gehören auch Stents aus dem sogenannten "Memory-Metall" Nitinol. Nitinol ist eine Nickel-Titanium-Legierung mit temperaturabhängigem Formverhalten. Wird beispielsweise einem Nitinoldraht eine bestimmte Form eingeprägt und anschließend der Draht über eine bestimmte "Memory-Temperatur" erhitzt, so gewinnt dieser Draht ein "Erinnerungsvermögen" für diese Form. Kühlt man anschließend den so behandelten Draht wieder unter seine Konversionstemperatur ab, die von der Legierung und der Wärmebehandlung abhängig ist, so wird er weich und leicht verformbar. Bei einer erneuten Erwärmung über die Konversionstemperatur nimmt der Draht automatisch die eingeprägte Form wieder an.

Selbstexpandierende Stents der eingangs genannten Art werden beispielsweise dadurch erzeugt, daß mit einem Laser in die Wand eines röhrenförmigen Körpers mit geringem Durchmesser parallel zu dessen Längsachse verlaufende Schlitze geschnitten werden. Diese Schlitze sind in Umfangsrichtung versetzt zueinander angeordnet, so daß bei einer Expansion des röhrenförmigen Körpers beispielsweise durch Balloninsufflation oder durch Erwärmung im Falle eines Stents aus Memory-Metall rautenförmige Durchbrechungen entstehen, deren Längsachsen ebenfalls parallel zur Längsachse des röhrenförmigen Körpers verlaufen.

Stents dieser Art haben jedoch den Nachteil, daß sie zum einen sowohl im komprimierten als auch im expandierten Zustand eine geringe Flexibilität aufweisen, so daß zum einen das Einführen in gebogene Hohlorgane nur in begrenztem Umfang möglich ist und zum anderen in Gelenkbereichen eingesetzte Stents zum Abknicken neigen, was zur Verringerung oder zum Unterbrechen des Blutflusses in Blutgefäßen oder gar zum Durchstoßen der Gefäßwand führen kann.

Darüber hinaus tritt durch die Expansion des Stents eine Verkürzung in Längsrichtung auf, die relativ unkontrolliert ist, so daß die Positionierung des Stents an einer bestimmten zu behandelnden Stelle innerhalb des Hohlorgans relativ schwierig sein kann.

Das Problem der geringen Flexibilität wird bei einem bekannten Stent dieser Art dadurch gelöst, daß die Verbindung zwischen einzelnen in Längsrichtung des Stents benachbart angeordneten Rauten unterbrochen wird. Nachteilig an dieser Anordnung ist jedoch, daß die freien, spitzen Rautenenden insbesondere bei einer Verbiegung des Stents, wie sie beispielsweise bei einer Kurvenimplantation erfolgt, am äußeren Kurvenradius aus der Wand des Stents herausragen und am inneren Kurvenradius in den Innenbereich des Stents hineinragen. Dies hat zur Folge, daß Verletzungen der Wand des Hohlorgans und des Ballons eines verwendeten Ballonkatheters auftreten können. Dies sind Komplikationen, die in der Praxis nicht hingenommen werden dürfen.

Der vorliegenden Erfindung liegt die Aufgabe zugrunde, einen Stent der eingangs genannten Art zu schaffen, der zum einen sowohl im komprimierten als auch im expandierten Zustand eine hohe Flexibilität aufweist, wobei gleichzeitig eine sichere, risikofreie Anwendung gewährleistet ist und bei dem durch die Expansion keine Verkürzung auftritt, so daß eine einwandfreie Positionierung des Stents erreichbar ist.

Diese Aufgabe wird nach der Erfindung ausgehend von einem Stent der eingangs genannten Art durch die kennzeichnenden Merkmale des Anspruchs 1 gelöst.

Überraschenderweise kann durch die schräge Anordnung der Durchbrechungen die Flexibilität des Stents sowohl im komprimierten als auch im expandierten Zustand gegenüber einem Stent mit im komprimierten Zustand parallel zur dessen Längsachse verlaufenden Durchbrechungen deutlich verbessert werden. Da somit auf das Durchtrennen der Verbindungen zwischen in Längsrichtung nebeneinander angeordneten Rauten verzichtet werden kann, existieren bei einem erfindungsgemäß ausgebildeten Stent keinerlei scharfe Kanten, die Verletzungen der Wand des Hohlorgans oder eines Ballons eines Ballonkatheters verursachen könnten.

Weiterhin wird durch die schräge Anordnung der Durchbrechungen erreicht, daß einer Verkürzungstendenz beim Expandieren des Stents entgegengewirkt werden kann. So wird beispielsweise bei der Vorbereitung eines selbstexpandierenden Stents aus Memory-Metall dieser aus seinem komprimierten Zustand durch Auffädeln auf eine Expansionsachse expandiert, wobei zunächst die übliche Verkürzung auftritt. Der Durchmesser der Expansionsachse wird dabei gleich dem gewünschten Durchmesser des Stents im expandierten Zustand gewählt.

Anschließend wird der auf die Expansionsachse aufgezogene Stent in Längsrichtung so weit gedehnt, bis die gewünschte Lange erreicht ist, wobei sich der Neigungswinkel eines Teils der schrägen Abschnitte der Durchbrechungen verringert. Wird der Stent in diesem Zustand Ober die Memory-Temperatur erhitzt, so nimmt er nach Abkühlen unter die Konversionstemperatur und anschließendem erneuten Erwärmen wiederum diese Form an, bei der die Verkürzung kompensiert ist. Üblicherweise entspricht die gewünschte Länge der Lange des Stents im komprimierten Zustand, um beim Einsetzen eine exakte Positionierung des expandierten Stents zu erreichen.

Nach einer vorteilhaften Ausführungsform der Erfindung bilden die Durchbrechungen im komprimierten Zustand des Stents in der Wand des röhrenförmigen Körpers schlitzförmige Öffnungen. Auf diese Weise ist eine besonders einfache Fertigung des erfindungsgemäß ausgebildeten Stents möglich, da die schlitzförmigen Öffnungen beispielsweise durch einen Laser in den röhrenförmigen Körper geschnitten werden können. Prinzipiell ist es jedoch auch möglich, daß die Ausnehmungen bereits im komprimierten Zustand breiter ausgebildet sind, was beispielsweise durch Stanz- oder Erodiervorgänge erreicht werden kann.

Die Durchbrechungen können sowohl im komprimierten als auch im expandierten Zustand des Stents erzeugt werden. Die Erzeugung im komprimierten Zustand ist jedoch vorteilhaft, da bei der Erzeugung der schlitzförmigen Öffnungen die Materialverluste geringer sind als bei der Erzeugung entsprechender expandierter Öffnungen.

Nach einer weiteren vorteilhaften Ausführungsform weisen die schlitzförmigen Öffnungen mehrere, insbesondere drei Abschnitte auf, welche zick-zack-förmig ausgebildet und jeweils schräg zur Längsachse des Stents angeordnet sind. Dadurch werden besonders gleichmäßige Biegeeigenschaften des Stents sowohl im komprimierten als auch im expandierten Zustand erreicht.

Das zwischen den Durchbrechungen liegende Material der Wand des röhrenförmigen Körpers bildet Umrandungselemente für die Durchbrechungen, die zur Expansion des Stents aufweitbar sind. Dabei sind die einander zugewandten Enden von in Längsrichtung benachbart angeordneten Umrandungselementen über insbesondere V-förmig ausgebildete Zwischenelemente verbunden. Durch diese Ausbildung wird zum einen eine noch flexiblere Ausgestaltung eines erfindungsgemäß ausgebildeten Stents erreicht. Zum anderen wird durch das Vorsehen von separaten Zwischenelementen eine Entkopplung zwischen den Umrandungselementen, welche in erster Linie die Expansion des Stents gewährleisten, und den Zwischenelementen, welche bevorzugt für die Verkürzungskompensation verwendet werden, erzielt.

Weitere vorteilhafte Ausführungsformen der Erfindung sind in den Unteransprüchen angegeben.

Im folgenden wird die Erfindung anhand eines Ausführungsbeispiels unter Bezugnahme auf die Zeichnungen näher beschrieben; in diesen zeigen:
- Fig. 1: ein verkürztes, flächig dargestelltes Schnittmuster zur Erzeugung der Durchbrechungen für einen erfindungsgemäßen Stent,
- Fig. 2: eine Detailansicht nach Fig. 1,
- Fig. 3: die Detailansicht nach Fig. 2 sowie eine dazu korrespondierende Detailansicht bei einem erfindungsgemäß ausgebildeten Stent im expandierten Zustand,
- Fig. 4: eine weitere Detailansicht eines erfindungsgemäß ausgebildeten Stents in radialer und tangentialer Richtung und
- Fig. 5: einen schematischen Querschnitt durch einen erfindungsgemäß ausgebildeten Stent.

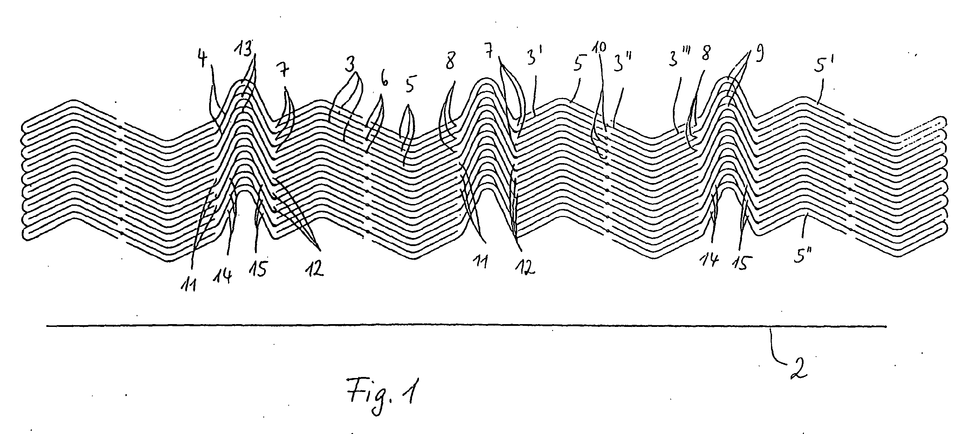

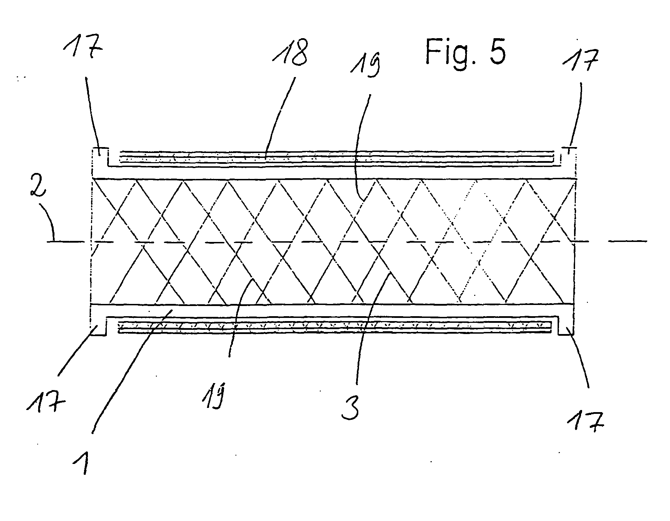

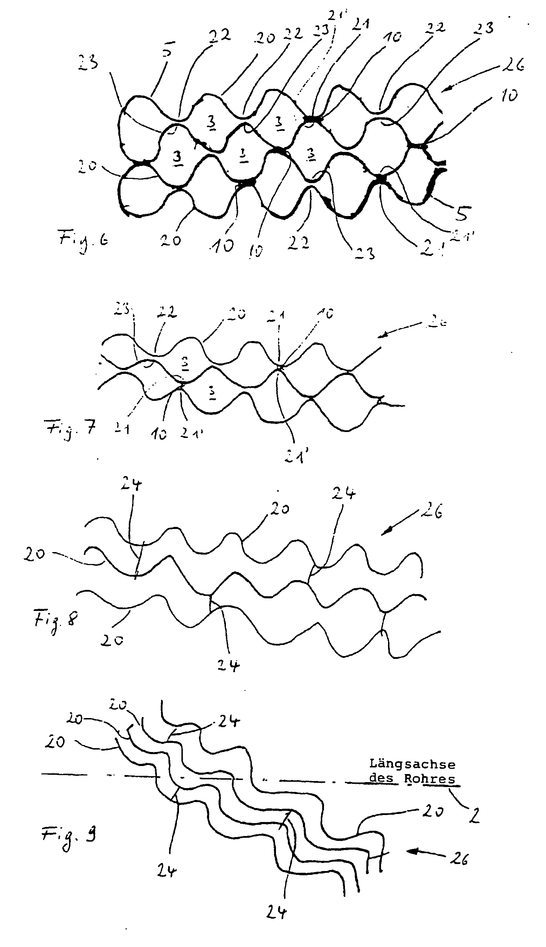

Fig. 1 zeigt ein Schnittmuster, wie es beispielsweise mittels eines Lasers in die Wand eines röhrenförmigen Körpers 1 (siehe Fig. 5) zur Herstellung eines erfindungsgemäß ausgebildeten Stents geschnitten wird. Zur Verdeutlichung der Lage der Schnitte innerhalb der Wand des röhrenförmigen Körpers 1 ist in Fig. 1 der Verlauf der Längsachse des röhrenförmigen Körpers 1 mit dem Bezugszeichen 2 versehen.

Durch den Schneidvorgang entstehen in der Wand des röhrenförmigen Körpers 1 Durchbrechungen 3, 4, die im komprimierten Zustand die Form der in Fig. 1 dargestellten schlitzförmigen Öffnungen besitzen.

Die schlitzförmigen Öffnungen 3 weisen drei Abschnitte 3', 3" und 3"' auf, die jeweils schräg zur Längsachse 2 des röhrenförmigen Körpers 1 angeordnet sind und zusammen jeweils Z-förmige schlitzförmige Öffnungen 3 bilden.

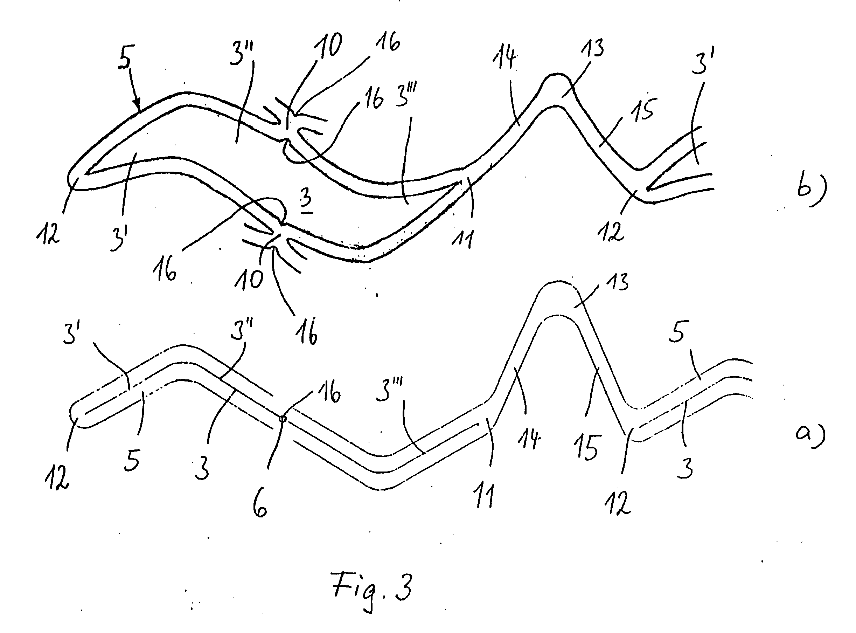

Das zwischen den Durchbrechungen 3 liegende Material der Wand des röhrenförmigen Körpers 1 bildet jeweils Umrandurtgselemente 5, die, wie besonders gut in Fig. 3b zu erkennen ist, jeweils Umgrenzungen für die Durchbrechungen 3 darstellen.

Die Durchbrechungen 3 sind punktsymmetrisch zu Symmetriepunkten 6 ausgebildet, die zusammen mit den jeweiligen Enden 7, 8 der Durchbrechungen 3 auf Parallelen zu der Längsachse 2 des röhrenförmigen Körpers 1 liegen. Der Symmetriepunkt 6 ist dabei in der Mitte zwischen den beiden Enden 7, 8 der Durchbrechungen 3 angeordnet.

Jeweils zwei in Umfangsrichtung des röhrenförmigen Körpers 1 benachbart angeordnete (in Fig. 1 übereinanderliegende) Umrandungselemente 5 sind über Verbindungsstellen 10 miteinander verbunden, welche jeweils zwischen den Symmetriepunkten 6 der Umrandungselemente 5 angeordnet sind.

Die einander zugewandten Enden 11, 12 von jeweils zwei in Längsrichtung benachbart angeordneten Umrandungselementen 5 sind jeweils über V-förmige Zwischenelemente 13 miteinander verbunden. Die Schenkel 14, 15 der Zwischenelemente 13 sind jeweils schräg zur Längsachse 2 des röhrenförmigen Körpers 1 angeordnet.

Die durch das in Fig. 1 dargestellte Schnittmuster erzeugten Durchbrechungen 3, 4 sind gleichmäßig über den gesamten Umfang des röhrenförmigen Körpers 1 verteilt, so daß beispielsweise die in Fig. 1 dargestellten Umrandungselemente 5' und 5'' zusammenfallen.

Je nach Länge des röhrenförmigen Körpers 1 können mehr oder weniger V-förmige Zwischenelemente 13 und Umrandungselemente 5 entlang der Längsachse 2 des röhrenförmigen Körpers 1 verteilt sein als in Fig. 1 dargestellt sind. Entsprechend kann je nach Umfang des röhrenförmigen Körpers 1 die Anzahl der V-förmigen Zwischenelemente 13 und der Umrandungselemente 5 entlang des Umfangs des röhrenförmigen Körpers 1 variieren.

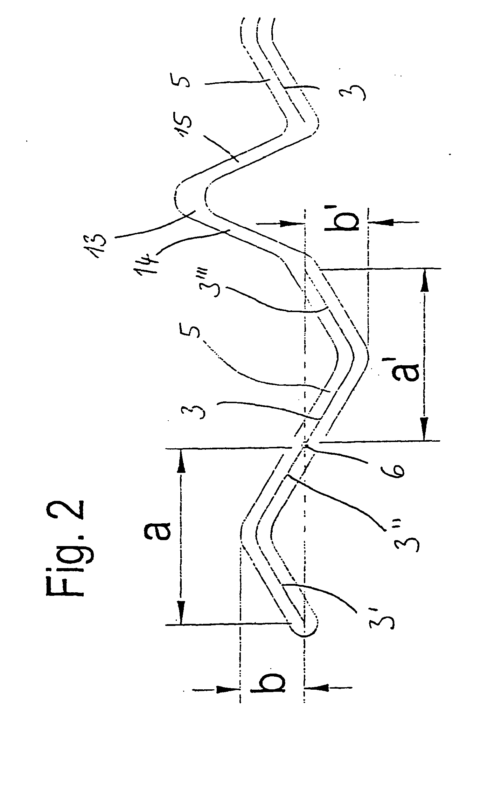

Aus Fig. 2 ist besonders deutlich der punktsymmetrische Aufbau der schlitzförmigen Öffnungen 3 und damit der Umrandungselemente 5 zu erkennen. Insbesondere werden die Vorteile eines erfindungsgemäßen Stents dadurch erreicht, daß die in Längsrichtung mit a und a' bzw. in Umfangsrichtung des röhrenförmigen Körpers 1 mit b und b' bezeichneten geometrischen Abstände jeweils gleich groß sind.

In Fig. 3 ist zu erkennen, wie durch Aufweiten der Durchbrechung 3 die Breite des Umrandungselementes 5 in Umfangsrichtung des röhrenförmigen Körpers 1 zunimmt, wodurch eine Expansion des röhrenförmigen Körpers 1 erreicht wird. Weiterhin ist Fig. 3 zu entnehmen, daß die auftretende Längsverkürzung des Umrandungselementes 5, die daran erkannt wird, daß das Ende 11 der Umrandung 5 in Fig. 3b gegenüber der Position in Fig. 3a nach links verschoben ist, durch ein gleichzeitiges Aufweiten des V-förmigen Zwischenelements 13 ausgeglichen wird, so daß die Positionen des Endes 12 des benachbart angeordneten Umrandungselements 5 im komprimierten (Fig. 3a) und im expandierten (Fig. 3b) Zustand übereinstimmen. Auf diese Weise besitzt jeweils eine Einheit aus Umrandungselement 5 und Zwischenelement 13 - und somit auch der röhrenförmige Körper 1 insgesamt - im komprimierten Zustand dieselbe Länge wie im expandierten Zustand.

Im Bereich der Verbindungsstellen 10 zwischen zwei in Umfangsrichtung des röhrenförmigen Körpers 1 benachbart angeordneten Rahmenelementen 5 sind Ausnehmungen 16 vorgesehen, so daß an diesen Stellen das Abbiegen der Umrandungselemente 5 erleichtert wird. Dadurch werden die elastische Eigenschaften eines erfindungsgemäßen Stents weiter verbessert.

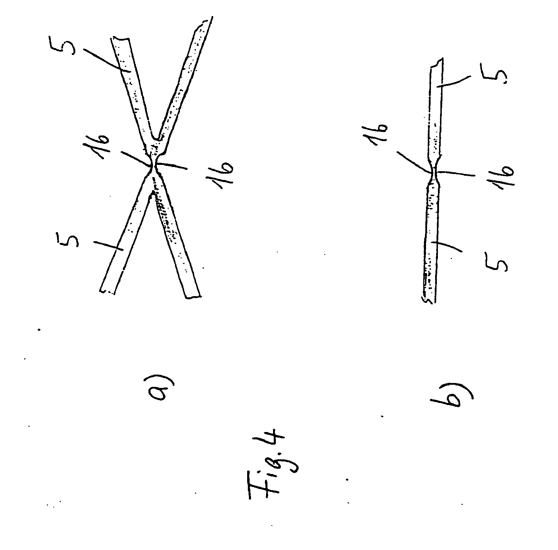

In Fig. 4 sind die Ausnehmungen 16 im Detail dargestellt, wie sie beispielsweise zwischen zwei unmittelbar miteinander verbundenen Umrandungselementen 5 vorgesehen sein können. Entsprechende Ausnehmungen 16 können auch zwischen den Umrandungselementen 5 und den V-förmigen Zwischenelementen 13 vorgesehen sein. Dabei sind sowohl Ausnehmungen in Umfangsrichtung, wie sie in Fig. 4a dargestellt sind, als auch Ausnehmungen in radialer Richtung, wie sie in Fig. 4b dargestellt sind, sowie jede weitere Art von Ausnehmungen möglich, die die Flexiblität des erfindungsgemäß ausgebildeten Stents günstig beeinflussen.

In dem in Fig. 5 schematisch dargestellten Querschnitt eines erfindungsgemäß ausgebildeten Stents 1 sind die Durchbrechungen 3 lediglich durch schräg verlaufende Striche 19 angedeutet. Der röhrenförmige Körper 1 weist an seinen beiden Enden jeweils in Umfangsrichtung verlaufende, als Vorsprünge 17 ausgebildete Verdickungen auf, welche sich radial nach außen erstrecken. Zwischen den Vorsprüngen 17 ist eine elastische Ummantelung 18 vorgesehen, deren radiale Stärke im wesentlichen gleich der radialen Abmessungen der überstehenden Teile der Vorsprünge 17 ist, so daß der Stent eine im wesentlichen gleichmäßige Außenfläche aufweist. Weiterhin ist aus Fig. 5 erkennbar, daß die Innenfläche des röhrenförmigen Körpers 1 gleichmäßig verlaufend ausgebildet ist.

Der erfindungsgemäß ausgebildete Stent wird bevorzugt wie folgt hergestellt, vorbereitet und verwendet:

In die Wand eines aus Memory-Metall bestehenden röhrenförmigen Körpers 1 werden mit einem Laser das in Fig. 1 dargestellte Schnittmuster und damit die schlitzförmigen Öffnungen 3, 4 geschnitten. Der Durchmesser des röhrenförmigen Körpers 1 wird dabei so gewählt, daß er dem zum Implantieren benötigten komprimierten Zustand des Stents entspricht.

Nachdem das in Fig. 1 dargestellte Schnittmuster über die gesamte Länge und den gesamten Umfang des röhrenförmigen Körpers 1 eingeschnitten wurde, wird der röhrenförmige Körper 1 auf eine Expansionsachse aufgezogen, deren Durchmesser, dem im eingesetzten, expandierten Zustand benötigten Durchmesser des Stents entspricht. Dadurch werden die schlitzförmigen Öffnungen 3 und 4 ausgeweitet, wie es in Fig. 3b dargestellt ist. Anschließend wird der auf die Expansionsachse aufgezogene röhrenförmige Körper in Längsrichtung soweit gedehnt, bis die durch die Expansion auftretende Verkürzung durch Aufbiegen der V-förmigen Zwischenelemente 13 kompensiert ist, so daß eine Oberflächenstruktur aus Umrandungselementen 5 und Zwischenelementen 13 entsteht, wie sie in Fig. 3b dargestellt sind.

Durch Erhitzen des röhrenförmigen Körpers über die Memory-Temperatur wird die entstandende Form anschließend in dem Material gespeichert.

Nach Abkühlen des Stents unter die Konversionstemperatur kann der Stent wieder auf seinen dem komprimierten Zustand entsprechenden Ausgangsdurchmesser zusammengedrückt werden und mit einer elastischen Ummantelung 18, die beispielsweise aus Nylon (eingetragenes Warenzeichen), Polyethylen, Polyamid oder Polyurethanelastomeren besteht, überzogen werden. Durch die Vorsprünge 17 wird ein versehendiches Abstreifen der elastischen Ummantelung 18 beim Implantieren verhindert. Gleichzeitig kann durch die Vorsprünge 17 der Stent beim Einsetzen über den Röntgenschirm besser beobachtet werden, so daß eine einwandfreie Positionierung des Stents an der gewünschten Stelle innerhalb des Hohlorgans gewährleistet ist.

Der Stent wird über einen Einführkatheter an der gewünschten Stelle positioniert, wobei ein Expandieren des Stents beispielsweise durch eine zusätzliche Umhüllung oder einen speziellen Katheter verhindert wird. Durch Abstreifen der Umhüllung oder des Katheters nimmt der röhrenförmige Körper 1 aufgrund der über der Konversionstemperatur liegenden Körpertemperatur seine gespeicherte Form an. Dabei stimmt aufgrund der durch die Zwischenelemente 13 erreichten Verkürzungskompensation die Länge im expandierten Zustand mit der Länge des Stents im komprimierten Zustand überein, so daß die beim Einsetzen über den Röntgenschirm beobachtete Position beider Enden des Stents eingehalten wird.

Aufgrund der erfindungsgemäßen Struktur des röhrenförmigen Körpers 1 wird sowohl im komprimierten als auch im expandierten Zustand eine hohe Flexibilität erreicht, so daß sowohl eine implantation in kurvenförmige Hohlorgane als auch in im Bereich von Gelenken angeordnete Hohlorgane möglich ist. Ein Abknicken des Stents durch Abbiegen der Gelenke wird durch die erzieke hohe Flexibilität weitgehend ausgeschlossen. Darüber hinaus ist durch die erfindungsgemäß ausgebildete Struktur sowohl im komprimierten als auch im expandierten Zustand eine gute Längs- und Querstabilität des Stents gewährleistet.

Weiterhin sind sowohl die Außenseite als auch die Innenseite des röhrenförmigen Körpers gleichmäßig ausgebildet und weisen insbesondere keine scharfkantigen nach außen oder innen hervorstehenden Elemente auf, so daß weder Verletzungen des Hohlorgans noch eines eventuell die Expansion unterstützenden Ballons eines Ballonkatheters auftreten können.

Außer durch die beschriebene Ausführungsform aus Memory-Metall können die Vorteile eines erfindungsgemäß ausgebildeten Stents auch bei Verwendung anderer Materialien, wie beispielsweise Tantal. Edelstahl oder körperverträglicher Kunststoffe, beispielsweise Polyethylen, Polyamid oder Polyurethanelastomere erreicht werden.

## Patentansprüche

1. Stent zur transluminalen Implantation in Hohlorgane, insbesondere in Blutgefäße, Harnleiter, Speiseröhren oder Gallenwege, mit einem im wesentlichen röhrenförmigen Körper (1), der von einem komprimierten Zustand mit einem ersten Querschnittsdurchmesser in einen expandierten Zustand mit einem vergrößerten zweiten Querschnittsdurchmesser überführbar ist, wobei die Wand des röhrenförmigen Körpers (1) sich sowohl in Längsrichtung als auch in Umfangsrichtung des Stents wiederholende, die Expansion gewährleistende Durchbrechungen (3, 4) aufweist, und wobei das zwischen den Durchbrechungen (3) liegende Material der Wand des röhrenförmigen Körpers Umrandungselemente für die Durchbrechungen (3) bildet,
**dadurch gekennzeichnet,**
**daß** jede von Umrandungselementen allseitig geschlossen umrandete Durchbrechung (3, 4) drei oder mehr längliche, jeweils in Längsrichtung des Stents hintereinanderliegend angeordnete Abschnitte (3', 3", 3"') aufweist, deren Längsachsen sowohl im komprimierten Zustand als auch im expandierten Zustand des Stents schräg zur Längsachse (2) des Stents angeordnet sind, und dass alle übrigen die Expansion gewährleistenden Durch brechnungen jeweils mehrere längliche Abschnitte aufweisen, deren Längsachsen sowohl im komprimierten als auch im expandierten Zustand des Stents schräg zur Längsachse des stents angeordnet sind.

2. Stent nach Anspruch 1,
**dadurch gekennzeichnet,**
**daß** die Durchbrechungen (3) im wesentlichen zick-zack-förmig, Z-förmig, S-förmig oder wellenförmig, insbesondere sinusförmig ausgebildet sind.

3. Stent nach einem der vorhergehenden Ansprüche,
**dadurch gekennzeichnet,**
**daß** die Neigung der Abschnitte (3', 3", 3'") zur Längsachse (2) des Stents im komprimierten Zustand jeweils zwischen 1° und 75°, insbesondere zwischen 10° und 45° beträgt.

4. Stent nach einem der vorhergehenden Ansprüche,
**dadurch gekennzeichnet,**
**daß** die Durchbrechungen (3) im wesentlichen punktsymmetrisch ausgebildet sind und/oder die beiden Enden (7, 8) der Durchbrechungen (3) auf einer Parallelen zur Längsachse (2) des Stents angeordnet sind, wobei insbesondere der Symmetriepunkt (6) auf der Parallelen zur Längsachse (2) des Stents in der Mitte zwischen den beiden Enden (7, 8) der Durchbrechungen (3) angeordnet ist.

5. Stent nach einem der vorhergehenden Ansprüche,
**dadurch gekennzeichnet,**
**daß** zwei in Umfangsrichtung benachbart angeordnete Umrandungselemente (5) insbesondere Ober einen Steg (10) miteinander verbunden sind und bevorzugt die Verbindungsstelle (10) jeweils im Bereich der Mitte zwischen den beiden Enden (11, 12) der Umrandungselemente (5) angeordnet ist.

6. Stent nach einem der vorhergehenden Ansprüche,
**dadurch gekennzeichnet,**
**daß** die einander zugewandten Enden (11, 12) von in Längsrichtung benachbart angeordneten Umrandungselementen (5) insbesondere Ober elastische, bevorzugt V-förmig ausgebildete Zwischenelemente (13) miteinander verbunden sind und die Zwischenelemente (13) insbesondere Abschnitte (14, 15) aufweisen, die schräg zur Längsachse (2) des Stents angeordnet sind.

7. Stent nach einem der Ansprüche 5 oder 6,
**dadurch gekennzeichnet,**
**daß** die Verbindungsstellen (10) zwischen den Umrandungselementen und/oder die Verbindungsstellen zwischen den Umrandungselementen und den Zwischenelementen in radialer und/oder axialer Richtung verjüngt ausgebildet sind.

8. Stent nach einem der vorhergehenden Ansprüche,
**dadurch gekennzeichnet,**
**daß** der röhrenförmige Körper (1) aus Memory-Metall, insbesondere aus Nitinol, und/oder aus körperverträglichem Kunststoff, insbesondere aus Polyethylen (PE), Polyamid (PA) oder Polyurerthanelastomeren (PUR) hergestellt ist.

9. Stent nach einem der vorhergehenden Ansprüche,
**dadurch gekennzeichnet,**
**daß** zumindest bin Ende des röhrenförmigen Körpers (1) eine insbesondere in Umfangsrichtung verlaufende Verdickung (17) aufweist die insbesondere nach außen, vorzugsweise in radialer Richtung nach außen übersteht

10. Stent nach einem der vorhergehenden Ansprüche,
**dadurch gekennzeichnet,**
**daß** an der Außenseite des röhrenförmigen Körpers (1) eine elastische Ummantelung (18) vorgesehen ist und/oder an jedem Ende des röhrenförmigen Körpers (1) eine nach außen überstehende Verdickung (17) vorgesehen ist und die elastische Ummantelung (18) zwischen den überstehenden Verdickungen (17) angeordnet ist, wobei bevorzugt die Materialstarke der elastischen Ummantelung (18) und die radialen Abmessungen der überstehenden Teile der Verdickungen (17) im wesentlichen gleich groß sind.

11. Stent nach einem der vorhergehenden Ansprüche,
**dadurch gekennzeichnet,**
**daß** die elastische Ummantelung (18) aus Kunststoff, insbesondere aus Polyethylen (PE), Polyamid (PA) oder Polyurethanelastomeren (PUR) besteht und/oder der Querschnittsdurchmesser des röhrenförmigen Körpers (1) zwischen 1 mm und 5 cm, insbesondere zwischen 3 mm und 3 cm liegt.

## Claims

1. Stent for the transluminal implantation in hollow organs, in particular in blood vessels, ureters, oesophagi or gall tracts, comprising a substantially tubular body (1) which can be transformed from a compressed state with a first cross-sectional diameter into an expanded state with a second enlarged cross-sectional diameter, wherein the wall of the tubular body (1) has apertures (3, 4) which repeat both in the longitudinal direction and also in the peripheral direction of the stent and ensure the expansion, and wherein the material of the wall of the tubular body disposed between the apertures (3) forms boundary elements for the apertures (3),
**characterized in that**
each bounded aperture (3, 4) closed on all sides by boundary elements has three or more elongate sections (3', 3", 3"') respectively arranged in series in the longitudinal direction of the stent, the longitudinal axes of which are arranged obliquely to the longitudinal axis (2) of the stent, both in the compressed state of the stent and also in the expanded state of the stent;
and **in that** all remaining apertures ensuring the expansion each have a plurality of elongate sections, the longitudinal axes of which are arranged obliquely to the longitudinal axis of the stent, both in the compressed state of the stent and also in the expanded state of the stent.

2. Stent in accordance with claim 1, **characterized in that** the apertures (3) are of substantially zigzag shape, of Z shape, of S shape or of wave shape, in particular of sinusoidal shape.

3. Stent in accordance with any one of the preceding claims, **characterized in that** the inclination of the sections (3', 3", 3"') to the longitudinal axis (2) of the stent in the compressed state respectively amount to between 1° and 75° and in particular to between 10° and 45°.

4. Stent in accordance with any one of the preceding claims, **characterized in that** the apertures (3) are made substantially point-symmetrical, and/or the two ends (7, 8) of the apertures (3) are arranged on a line parallel to the longitudinal axis (2) of the stent, wherein the point of symmetry (6) is in particular arranged on a line parallel to the longitudinal axis (2) of the stent at the centre between the two ends (7, 8) of the apertures (3).

5. Stent in accordance with any one of the preceding claims, **characterized in that** two boundary elements (5) which are arranged adjacent to one another in the circumferential direction are connected to one another, in particular via one web (10), and the connection location (10) is preferably disposed in each case in the region of the centre between two ends (11, 12) of the boundary elements (5).

6. Stent in accordance with any one of the preceding claims, **characterized in that** the confronting ends (11, 12) of boundary elements (5) which are arranged adjacent to one another in the longitudinal direction are connected to one another in particular via resilient, preferably V-shaped intermediate elements (13), and the intermediate elements (13) in particular have sections (14, 15) which are arranged obliquely to the longitudinal axis (2) of the stent.

7. Stent in accordance with claim 5 or claim 6, **characterized in that** the connection locations (10) between the boundary elements and/or the connection locations between the boundary elements and the intermediate elements are tapered in the radial and/or axial direction.

8. Stent in accordance with any one of the preceding claims, **characterized in that** the tubular body (1) is manufactured from memory material, in particular from Nitonol, and/or from a plastic compatible with the body, in particular from polyethylene (PE), polyamide (PA) or polyurethane elastomers (PUR).

9. Stent in accordance with any one of the preceding claims, **characterized in that** at least one end of the tubular body (1) has a thicker portion (17) extending in particular in the circumferential direction, and which in particular projects outwardly and preferably projects outwardly in the radial direction.

10. Stent in accordance with any one of the preceding claims, **characterized in that** a resilient jacket (18) is provided at the outer side of the tubular body (1), and/or that an outwardly projecting thicker portion (17) is provided at each end of the tubular body (1) and the resilient jacket (18) is arranged between the projecting thicker portions (17), wherein the material thickness of the resilient jacket (18) and the radial dimensions of the projecting parts of the thicker portion (17) are preferably of substantially the same size.

11. Stent in accordance with any one of the preceding claims, **characterized in that** the resilient jacket (18) consists of plastic, in particular of polyethylene (PE), polyamide (PA) or polyurethane elastomers (PUR), and/or **in that** the cross-sectional diameter of the tubular body (1) lies between 1 mm and 5 cm, in particular between 3 mm and 3 cm.

## Revendications

1. Stent pour implantation transluminale dans des organes creux, en particulier dans des vaisseaux sanguins, des voies urinaires, des oesophages ou des voies biliaires, comprenant un corps essentiellement tubulaire (1) qui est susceptible de passer d'un état comprimé avec un premier diamètre de section transversale jusque dans un état dilaté avec un second diamètre de section transversale agrandi, la paroi du corps tubulaire (1) présentant des traversées (3, 4) qui se répètent aussi bien en direction longitudinale qu'en direction périphérique du stent, qui assurent la dilatation, et le matériau de la paroi du corps tubulaire (1) situé entre les traversées (3) forme des éléments d'entourage pour les traversées (3),
**caractérisé en ce que**
chaque traversée (3, 4) entourée et fermée de tous côtés par des éléments d'entourage comporte trois ou plusieurs tronçons oblongs (3', 3", 3"'), dont les axes longitudinaux sont agencés en oblique par rapport à l'axe longitudinal (2) du stent, aussi bien dans l'état comprimé que dans l'état dilaté, et **en ce que** toutes les traversées restantes assurant la dilatation comprennent chacune plusieurs tronçons oblongs, dont les axes longitudinaux sont agencés en oblique par rapport à l'axe longitudinal (2) du stent, aussi bien dans l'état comprimé que dans l'état dilaté.

2. Stent selon la revendication 1, **caractérisé en ce que** les traversées (3) sont réalisées essentiellement en forme de zigzag, en forme de Z, en forme de S ou en forme ondulée, en particulier en forme sinusoïdale.

3. Stent selon l'une des revendications précédentes, **caractérisé en ce que** la pente des tronçons (3', 3", 3"') par rapport à l'axe longitudinal (2) du stent à l'état comprimé s'élève respectivement entre 1° et 75°, en particulier entre 10° et 45°.

4. Stent selon l'une des revendications précédentes, **caractérisé en ce que** les traversées (3) sont réalisées sensiblement sous forme à symétrie ponctuelle et/ou **en ce que** les deux extrémités (7, 8) des traversées (3) sont agencées sur une parallèle à l'axe longitudinal (2) du stent, et le point de symétrie (6) est agencé en particulier sur la parallèle à l'axe longitudinal (2) du stent au milieu entre les deux extrémités (7, 8) des traversées (3).

5. Stent selon l'une des revendications précédentes, **caractérisé en ce que** deux éléments d'entourage agencés adjacents en direction périphérique sont reliés l'un à l'autre, en particulier par l'intermédiaire d'une barrette (10), et **en ce que** l'emplacement de liaison (10) est de préférence agencé respectivement dans la région du milieu entre les deux extrémités (11, 12) des éléments d'entourage (5).

6. Stent selon l'une des revendications précédentes, **caractérisé en ce que** les extrémités (11, 12) tournées l'une vers l'autre des éléments d'entourage (5) agencés adjacents en direction longitudinale, sont reliées l'une à l'autre en particulier par des éléments intermédiaires élastiques (13), réalisés de préférence en forme de V, et **en ce que** les éléments intermédiaires (13) présentent en particulier des tronçons (14, 15) qui sont agencés en oblique par rapport à l'axe longitudinal (2) du stent.

7. Stent selon l'une ou l'autre des revendications 5 et 6, **caractérisé en ce que** les emplacements de liaison (10) entre les éléments d'entourage et/ou les emplacements de liaison entre les éléments d'entourage et les éléments intermédiaires sont réalisés de manière à se rétrécir en direction radiale et/ou en direction axiale.

8. Stent selon l'une des revendications précédentes, **caractérisé en ce que** le corps tubulaire (1) est produit en métal à effet mémoire, en particulier en Nitinol, et/ou en matière plastique compatible avec le corps, en particulier en polyéthylène (PE), en polyamide (PA) ou en élastomère au polyuréthane (PUR).

9. Stent selon l'une des revendications précédentes, **caractérisé en ce que** l'une au moins des extrémités du corps tubulaire (1) comporte un épaississement (17) qui s'étend en particulier en direction périphérique, et qui dépasse en particulier vers l'extérieur, et dépasse de préférence vers l'extérieur en direction radiale.

10. Stent selon l'une des revendications précédentes, **caractérisé en ce que** sur la face extérieure du corps tubulaire (1) est prévue une enveloppe élastique (18) et/ou **en ce qu'**il est prévu à chaque extrémité du corps tubulaire (1) un épaississement (17) qui dépasse vers l'extérieur, et **en ce que** l'enveloppe élastique (18) est agencée entre les épaississements en dépassement (17), et l'épaisseur de matériau de l'enveloppe élastique (18) et les dimensions radiales des parties en dépassement des épaississements (17) sont de préférence égales.

11. Stent selon l'une des revendications précédentes, **caractérisé en ce que** l'enveloppe élastique (18) est réalisée en matière plastique, en particulier en polyéthylène (PE), en polyamide (PA) ou en élastomère au polyuréthane (PUR) et/ou **en ce que** le diamètre de section transversale du corps tubulaire (1) est compris entre 1 mm et 5 cm, en particulier entre 3 mm et 3 cm.
